(19) **Europäisches Patentamt**
**European Patent Office**
**Office européen des brevets**

(11) **EP 4 474 820 B1**

(12) **EUROPEAN PATENT SPECIFICATION**

(45) Date of publication and mention
of the grant of the patent:
**08.07.2026 Bulletin 2026/28**

(21) Application number: **24177719.2**

(22) Date of filing: **23.05.2024**

(51) International Patent Classification (IPC):
***G01N 33/38*** (2006.01)

(52) Cooperative Patent Classification (CPC):
**G01N 33/383;** G01N 2223/079; G01N 2223/418;
G01N 2223/605; G01N 2223/616

(54) **METHOD OF DETERMINING THE TYPE OF MASONRY MORTAR**

VERFAHREN ZUR BESTIMMUNG DES TYPS VON MAUERMÖRTEL

PROCÉDÉ POUR DÉTERMINER LE TYPE DE MORTIER DE MAÇONNERIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **05.06.2023 PL 44512223**

(43) Date of publication of application:
**11.12.2024 Bulletin 2024/50**

(73) Proprietor: **Instytut Techniki Budowlanej
00-611 Warszawa (PL)**

(72) Inventor: **Chylinski, Filip
Dziekanow Lesny (PL)**

(74) Representative: **AOMB Polska Sp. z.o.o.
ul. Rondo Ignacego Daszynskiego 1
00-843 Warsaw (PL)**

(56) References cited:
• HE H ET AL: "The effect of grain size on Gmaxof a demolished structural concrete: A study through energy dispersive spectroscopy analysis and dynamic element testing", SOIL DYNAMICS AND EARTHQUAKE ENGINEERING, vol. 89, 17 August 2016 (2016-08-17), pages 208 - 218, XP029734643, ISSN: 0267-7261, DOI: 10.1016/J.SOILDYN.2016.08.011
• FREIRE MARIA TERESA ET AL: "Studies in ancient gypsum based plasters towards their repair: Mineralogy and microstructure", CONSTRUCTION AND BUILDING MATERIALS, vol. 196, 23 November 2018 (2018-11-23), pages 512 - 529, XP085574890, ISSN: 0950-0618, DOI: 10.1016/J.CONBUILDMAT.2018.11.037

## Description

### Field of invention

The invention relates to a method of determining the type of masonry mortar. More specifically, the invention relates to the application of scanning microscopy with composition analysis in a SEM-EDX micro-area in the determination of the type of masonry mortar from cement, cement-lime and lime mortars.

### State of the art

Mortars are binders used to bond small-sized masonry units into a structure. Masonry mortar consists mainly of fine aggregate (most often sand), mineral binder and water. In accordance with the European standard PN-EN 998-2:2016-12 *Specification for mortar for masonry - Part 2: Masonry mortar masonry* mortar is a mixture containing one or more types of mineral binders, aggregate and water and may also contain admixtures and/or additives. The types of mineral binders used can be divided into three main groups: lime, cement and cement-lime, in accordance with the standard PN-B-10104 *Requirements for masonry mortars of general use - Prescribed masonry mortars, mixed on building site.* In each of these groups, further subgroups can be distinguished including additional characteristics of the binder, for example, different types of lime, cements, and the use of active additives. During repair works, in particular the renovation of historic buildings, it is necessary to use mortar of the same type as the original mortar.

Distinguishing the type of binder used, for example, cement mortar from cement-lime mortar is a challenge due to the fact that the range of proportions of components used is very wide, because they are most often mixed by hand on the construction site, and the hydration products that can be identified in samples of hardened mortars are similar, for example, portlandite, calcite, C-S-H phase and clinker relics. In addition, the identification is hindered by the use of sand from various deposits characterized by diverse composition.

The type of masonry mortar used can often be determined by measuring its compressive strength, but the test method requires that the test samples have dimensions of 40 x 40 x 160 mm or greater, while the thickness of the joint in the masonry rarely exceeds 10 mm, and besides, the process of sampling of hardened mortar for test itself affects their subsequent strength.

Mortar samples that can be taken from the building structure make it impossible to carry out most of strength tests in accordance with the relevant standards. The only possibility of assessing the mortar can be found in the analysis of its composition and microstructure.

The results of testing the elemental composition of the entire mortar, for example by X-ray fluorescence spectroscopy (XRF), may not provide conclusive information on the type of mortar. This is due to the fact that the elements Si, Ca, Al most abundantly present in the composition of the mortar are both components of the binder and aggregate.

Therefore, development of a research method allowing one to determine the type of masonry mortar became necessary.

In the current state of the art, solutions for the application of the scanning microscopy technique with SEM-EDX micro-area analysis in the study of the composition of masonry mortar are known.

In Klimek, B. "Badania historycznych zapraw z Baszty Gotyckiej w Lublinie., TEKA Komisji Architektury, Urbanistyki i Studiów Krajobrazowych Oddzial Polskiej Akademii Nauk w Lublinie 2022, 18(3), pp. 18-29", analyses of the elemental composition on fracture samples in individual, selected points of the masonry mortar collected from the Gothic Tower in Lublin were presented. The type of masonry mortar was not examined in the document. In addition, no research was carried out using the methodology consisting in the study of polished microsections with the analysis of the elemental composition of binder mass areas without taking into account aggregate grain and the subsequent establishment of the Si/Ca ratio. In Stomka-Stupik B., et al., *"Diagnostyczne badania wykwitów solnych na restaurowanych elewacjach Cz<sup>eść</sup>* 2 - *badania zapraw i tynków.", Ochrona przed Korozją 2018, 61(2), pp.* 43-47, the SEM-EDX technique was used to determine the elemental composition at selected points of the analysed fractures in the samples collected, including mortar samples. The document did not address the issue of identifying the type of masonry mortar used from among lime, cement-lime, or cement mortar.

At the Wroctaw University of Technology, Faculty of Architecture - Technical and Conservation Laboratory, "WYNIKI BADAŃ PROBEK ZAPRAW Badania w ramach projektu badawczego pn.: Cmentarz Salvatora - pierwsza nekropolia wroctawskich protestantów" Series: W1/ 430-3/2014, June 2014, the results of tests of mortar samples were presented. The scope of the research also included microstructural studies using the SEM-EDX technique. The research presented in the document was limited to the observation of the microstructure of mortar fractures and point analysis of the elemental composition in selected sites of the areas under study. The microscopic observations presented are typical studies performed using SEM-EDX.

H. He and K. Senetakis, Soil Dynamics and Earthquake Engineering, vol. 89 (2016), pages 208-218, disclose analysing samples of recycled concrete aggregates, which comprise cement mortar and aggregate components, by determining their Si/Ca ratios using SEM-EDS.

## Subject matter of the invention

The subject matter of the invention is a method for determining the type of masonry mortar, by establishing the ratio of atomic Si/Ca as percentage points in the tested sample, including the following stages: mechanical collection of a mortar fragment, drying the sample at a temperature not lower than 40 °C for at least 8 hours, then flooding the cooled sample with epoxy resin under reduced pressure, exposing the surface of the mortar cross-section by grinding and sputtering it with a conductive material selected from a group including gold, platinum, palladium, osmium, and carbon, with the sputtering layer being at least 1 nm; determining the Si/Ca ratio based on the Si and Ca content by microscopic examination of the sample using a scanning microscope using a voltage of at least 10 kV and an aperture of at least 1 $\mu$m; the microscopic examination of the prepared sample is the very beginning of the sample, omitting the aggregate grain on the SEM scanning electron microscope integrated with the X-ray energy dispersion spectrometer EDX.

The method specified by the invention, unlike other methods, allows for accurate determination of the type of masonry mortar under study. Limiting the research to the area of the binder mass alone, omitting aggregate grains, has a positive effect on the accuracy of the results obtained. Such information may, among other things, assist in recreating mortar for fillings used in restoration works.

## Brief description of the drawings

The subject matter of the invention is illustrated in examples of its execution shown the drawing, in which:

> Fig. 1. shows an example image of a sample of cement mortar;
> Fig. 2. shows an analysis of the elemental composition of the binder mass area indicated in Fig. 1. with a white line;

## Embodiments of the invention

The test samples may be fragments of mortar collected from a masonry structure or another element, mechanically, for example by cutting out or drilling cores. After sampling, the samples are prepared for microscopic examinations.

## Embodiment 1. Preparation of test samples for microscopic examination.

The cut-out mortar fragment with a volume of 1cm$^3$ was dried at a temperature of 40 °C for 8 hours. Depending on the size of the sample, the drying stage may proceed at a higher temperature and for more than 8 hours. Then, after cooling, it was immersed in epoxy resin under vacuum. After the epoxy resin had hardened, the sample was subjected to grinding and polishing in order to reveal an even and smooth cross-sectional surface of the mortar. Immediately before performing observation using a scanning microscope, the sample was sputtered with conductive material, for example gold, platinum, palladium, osmium or carbon. The thickness of the sputtered layer is at least 1 nm.

## Embodiment 2. Examination of the sample prepared for microscopic examinations.

The prepared mortar samples are examined using a scanning microscope, and the elemental composition of the areas containing the binder mass alone is analysed, excluding aggregate grains. The analysis of the composition is limited only to the content of Ca, Si and Al. The tests are carried out in several randomly selected areas of the sample under study. It is crucial that all analyses are carried out using the same equipment, at the same magnification and using the same exposure parameters, including accelerating voltage, aperture, working distance of the sample from the electron gun.

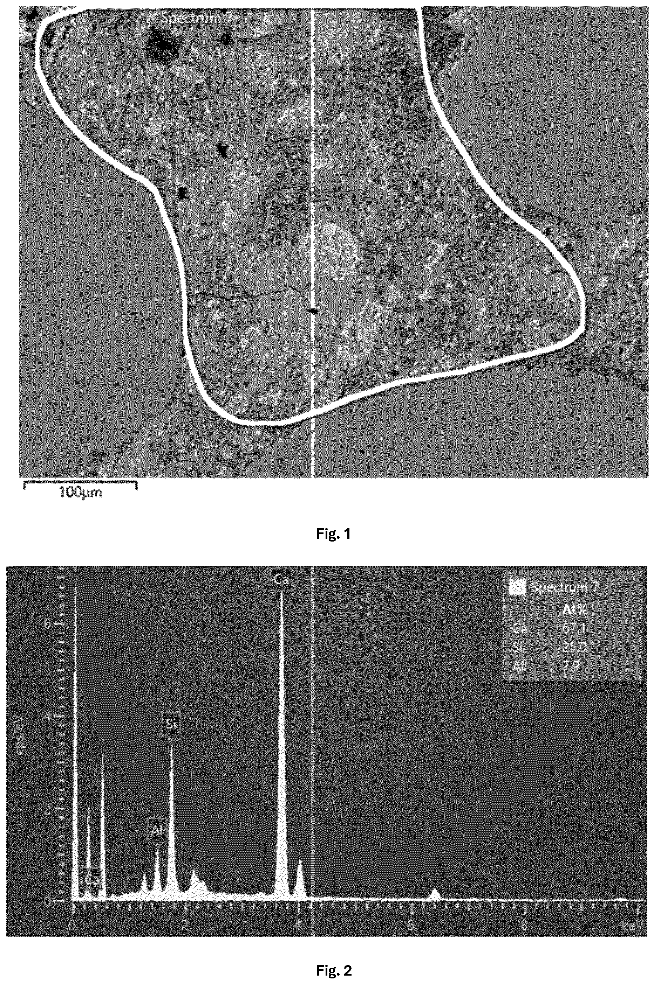

The prepared sample of cement mortar was subjected to microscopic examination. The sample was analysed at a constant magnification of at least 500x, an accelerating voltage of 15 kV and an aperture of 30 $\mu$m. The acceleration voltage should be at least 10 kV and the aperture at least 1 $\mu$m. An example image and the result of the analysis of the binder mass composition are shown in Fig. 1 and Fig. 2.

The examination of the binder mass composition was repeated in ten randomly selected areas of the sample, recording the content of Ca, Si, Al in atomic percent figures. The values obtained were averaged obtaining the following content figures:

$$Ca = 64.82\%$$

$$Si = 27.62\%$$

$$Al = 7.56\%$$

On the basis of the collected research results, the content ratios of Si/Ca, Al/Ca and Al/Si are determined, and it was found that the most useful ratio for determining the type of mortar is Si/Ca. Based on a series of analyses of various standard mortars with a known composition, the range of the value of the Si/Ca ratio for each type of masonry mortars including cement, cement-lime and lime mortars was determined.

The tests of standard mortars of a known composition, including cement, cement-lime and lime mortars, were carried out and the average content of Ca, Si, Al was determined for each of the standard mortars with the same observation parameters. The Si/Ca ratio was determined for each of the mortars. It was found that the value of the Si/Ca ratio determines the type of mortar in the following manner:

Si/Ca > 0.40 for cement mortars,

0.40 ≤ Si/Ca < 0.20 for cement-lime mortars,

Si/Ca ≤ 0.20 for lime mortars.

Analysis of an unknown mortar type was performed. The value of the Si/Ca ratio = 0.32 was obtained. Based on the above classification, it was established that the mortar under study was a cement-lime mortar.

## Claims

1. A method of determining the type of masonry mortar, by establishing the atomic ratio for Si/Ca in the tested sample, including the following stages:

   a) mechanical collection of a mortar fragment,
   b) drying the sample from stage a) at a temperature not lower than 40 °C for at least 8 hours, then immersing the cooled sample in epoxy resin under vacuum, exposing the surface of the mortar cross-section by grinding and polishing and sputtering with a conductive material selected from a group including gold, platinum, palladium osmium and carbon, with the thickness of the layer formed by sputtering being at least 1 nm;
   c) determining the Si/Ca ratio based on the Si and Ca content by microscopic examination of the sample from stage b) using a scanning microscope at an accelerating voltage of at least 10 kV and an aperture of at least 1 μm;

   wherein the microscopic examination of the prepared sample consists in examining the binder mass in the sample alone, omitting aggregate grains, using an SEM scanning electron microscope integrated with the EDX X-ray energy dispersion spectrometer.

## Patentansprüche

1. Verfahren zur Bestimmung der Art von Mauermörtel durch Festlegung des Atomverhältnisses von Si/Ca in der untersuchten Probe, umfassend die folgenden Schritte:

   a) mechanische Entnahme eines Mörtelfragments,
   b) Trocknen der Probe aus Schritt a) bei einer Temperatur von nicht weniger als 40 °C für mindestens 8 Stunden, anschließend Einbetten der abgekühlten Probe in Epoxidharz unter Vakuum, Freilegen der Oberfläche des Mörtelquerschnitts durch Schleifen und Polieren sowie Bedampfen mit einem leitfähigen Material, ausgewählt aus einer Gruppe umfassend Gold, Platin, Palladium, Osmium und Kohlenstoff, wobei die Dicke der durch das Bedampfen gebildeten Schicht mindestens 1 nm beträgt;
   c) Bestimmung des Si/Ca-Verhältnisses auf Grundlage des Si- und Ca-Gehalts durch mikroskopische Untersuchung der Probe aus Schritt b) unter Verwendung eines Rastermikroskops bei einer Beschleunigungsspannung von mindestens 10 kV und einer Apertur von mindestens 1 μm;

   **dadurch gekennzeichnet, dass** die mikroskopische Untersuchung der vorbereiteten Probe darin besteht, ausschließlich die Bindemittelmasse in der Probe unter Auslassung der Zuschlagstoffkörner unter Verwendung eines mit einem EDX-Röntgenenergiedispersionsspektrometer integrierten SEM-Rasterelektronenmikroskops zu untersuchen.

## Revendications

1. Procédé de détermination du type de mortier de maçonnerie par établissement du rapport atomique Si/Ca dans l'échantillon testé, comprenant les étapes suivantes :

   a) prélèvement mécanique d'un fragment de mortier,
   b) séchage de l'échantillon de l'étape a) à une température non inférieure à 40 °C pendant au moins 8 heures, puis immersion de l'échantillon refroidi dans une résine époxy sous vide, exposition de la surface de la section transversale du mortier par meulage et polissage et pulvérisation d'un matériau conducteur choisi dans un groupe comprenant l'or, le platine, le palladium, l'osmium et le carbone, l'épaisseur de la couche formée par pulvérisation étant d'au moins 1 nm ;
   c) détermination du rapport Si/Ca sur la base de la teneur en Si et Ca par examen microscopique de l'échantillon de l'étape b) au moyen d'un microscope à balayage à une tension d'accélération d'au moins 10 kV et une ouverture d'au moins 1 μm ;

   **caractérisé en ce que** l'examen microscopique de l'échantillon préparé consiste à examiner uniquement la masse de liant dans l'échantillon, en omettant les grains d'agrégat, au moyen d'un microscope électronique à balayage SEM intégré à un spectromètre de dispersion d'énergie des rayons X EDX.

EP 4 474 820 B1

**Fig. 1**

**Fig. 2**

5

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KLIMEK, B**. Badania historycznych zapraw z Baszty Gotyckiej w Lublinie.. TEKA Komisji Architektury, Urbanistyki i Studiów Krajobrazowych Oddzial Polskiej Akademii Nauk w Lublinie, 2022, vol. 18, 18-29

- **H. HE** ; **K. SENETAKIS**. *Soil Dynamics and Earthquake Engineering*, 2016, vol. 89, 208-218